# EUROPEAN PATENT APPLICATION

(11) **EP 3 295 921 A1**
(43) Date of publication of application: **21.03.2018**
(21) Application number: 17190467.5
(22) Date of filing: 11.09.2017
(51) Int. Cl.: A61K 8/24, A61K 8/25, A61Q 11/00, A61K 8/19

(54) **USE OF ORAL CARE COMPOSITION**

(30) Priority: 14.09.2016 EP 16188788
(71) Applicant: Unilever PLC, London, Greater London EC4Y 0DY (GB); Unilever N.V., 3013 AL Rotterdam (NL)
(72) Inventor: CHOPRA, Anita, Bebington, Wirral, Merseyside CH63 3JW (GB); COLLINS, Luisa, Zoe, Bebington, Wirral, Merseyside CH63 3JW (GB)
(74) Representative: Tansley, Sally Elizabeth

(57) **Abstract**

The invention provides the use of an oral care composition to enhance the behaviours associated with attractiveness of an individual.

The present invention is concerned with oral care products that after use can bring about an immediate change in altered non-verbal behaviour, reflecting increasing confidence and attractiveness that is detected by third party viewers.

## Description

The present application relates to how oral care compositions can affect interpersonal behaviour.

### Background

Teeth cleaning and the associated oral hygiene is claimed by toothpastes to increase self-confidence. This approach is unsurprising given the positive association of oral health with self esteem, confidence, and first impressions. The present invention is concerned with oral care products that after use can bring about an immediate change in altered non-verbal behaviour, reflecting increasing confidence and attractiveness that is detected by third party viewers.

### Description of the Invention

The present invention relates to use of an oral care composition to enhance the behaviours associated with attractiveness of an individual.

### Detailed Description of the Invention

Oral care compositions, preferably mouthwashes, more preferably toothpastes, most preferably gel toothpastes when used to clean the teeth can enhance the behaviours associated with attractiveness of an individual.

This effect is particularly enhanced when the oral care composition is used to clean the teeth within two hours, preferably one hour most preferably half an hour from when the behaviour is assessed.

Preferably, the behaviour associated with attractiveness is non-verbal behaviour, more preferably it is increased eye gaze. It is preferable if the breath is not assessed, it is also preferable if the behaviour is purely based on non-verbal behaviour and no other behaviour is taken into account.

In a preferred assessment, the behaviour is assessed for attractiveness by a third party and is not self-assessed.

Compositions of the invention preferably comprise an abrasive. Preferably, the abrasive is selected from abrasive silica, calcium carbonate, calcium pyrophosphate, calcined alumina, tungsten carbide, perlite, silicon carbide, polypropylene, polycarbonate, dicalciumphosphates, hydroxyapatites, trimetaphosphates, insoluble hexametaphosphates and mixtures thereof. More preferably, the abrasive is abrasive silica, perlite, silicon carbide or tungsten carbide. Most preferably, it is a silica based abrasive such as AC33 available from Ineos Silicas.

Compositions of the invention comprise a liquid phase. Preferred abrasives include any that have a Refractive Index matched to the Refractive Index of the liquid phase of the composition. These are commonly known as the low refractive index silicas. The low refractive index silicas used as abrasives in the present invention are silicas which have a refractive index which is matched to that of the aqueous sorbitol base with an apparent refractive index in the range of 1.41 -1.47, preferably 1.435 -1.445, preferably having a weight mean particle size of between 5 and 15 µm, a BET (nitrogen) surface area of between 10 and 100 m²/g and an oil absorption of about 70 - 150 cm³/100 g.

Typical examples of suitable low refractive index abrasive silicas having an R.I. of between 1.435 and 1.445 are Tixosil 63 and 73 ex Rhone Poulenc; Sident 10 ex Degussa; Zeodent 113 ex Zeofinn; Sorbosil AC 77 ex Ineos having an R.I. of approximately 1.440. Further examples can be found in EP-A-0,549,287 (Colgate) and in WO 94/10087 (Crosfield) which are hereby incorporated by way of Reference. The amount of these silicas in the composition generally ranges from 5-60% by weight, usually 5-20% by weight.

The liquid phase of the formulation should be formulated such, that its refractive index closely matches the refractive index of the silicas as discussed above. The liquid phase usually comprises water and a humectant, the latter usually being sorbitol or glycerol. Water has an RI of 1.333, sorbitol (70% aqueous solution) has an RI of 1.457 and glycerine an RI of 1.473. Other humectants such as propylene glycol, polypropylene glycol and polyethylene glycol may also be present. The refractive index of the liquid phase can be calculated from the respective refractive indices of the constituting ingredients. The most preferred abrasive of this type is AC77 available from Ineos Silicas.

The oral composition according to the invention comprise further ingredients which are common in the art, such as: antimicrobial agents, e.g. Triclosan, chlorhexidine, copper-, zinc- and stannous salts such as zinc citrate, zinc sulphate, zinc glycinate, sodium zinc citrate and stannous pyrophosphate, sanguinarine extract, metronidazole, quaternary ammonium compounds, such as cetylpyridinium chloride; bis-guanides, such as chlorhexidine digluconate, hexetidine, octenidine, alexidine; and halogenated bisphenolic compounds, such as 2,2' methylenebis-(4-chloro-6-bromophenol);
anti-inflammatory agents such as ibuprofen, flurbiprofen, aspirin, indomethacin etc.; anti-caries agents such as sodium- and stannous fluoride, aminefluorides, sodium monofluorophosphate, sodium trimeta phosphate and casein;plaque buffers such as urea, calcium lactate, calcium glycerophosphate and strontium polyacrylates; vitamins such as Vitamins A, C and E; plant extracts; desensitising agents, e.g. potassium citrate, potassium chloride, potassium tartrate, potassium bicarbonate, potassium oxalate, potassium nitrate and strontium salts; anti-calculus agents, e.g. alkali-metal pyrophosphates, hypophosphite-containing polymers, organic phosphonates and phosphocitrates etc.; biomolecules, e.g. bacteriocins, antibodies, enzymes, etc.; flavours, e.g. peppermint and spearmint oils; proteinaceous materials such as collagen; preservatives; opacifying agents; colouring agents; pH-adjusting agents; sweetening agents; pharmaceutically acceptable carriers, e.g. starch, sucrose, water or water/alcohol systems etc.; surfactants, such as anionic, nonionic, cationic and zwitterionic or amphoteric surfactants; humectants such as glycerol, sorbitol, propyleneglycol, xylitol, lactitol etc.; binders and thickeners such as sodium carboxymethyl-cellulose, xanthan gum, gum arabic etc. as well as synthetic polymers such as polyacrylates and carboxyvinyl polymers such as Carbopol®; polymeric compounds which can enhance the delivery of active ingredients such as antimicrobial agents can also be included. Examples of such polymers are copolymers of polyvinylmethylether with maleic anhydride and other similar delivery enhancing polymers, e.g. those described in DE-A-3,942,643 (Colgate); buffers and salts to buffer the pH and ionic strength of the oral care composition; and other optional ingredients that may be included are e.g. bleaching agents such as peroxy compounds e.g. potassium peroxydiphosphate, effervescing systems such as sodium bicarbonate/citric acid systems, colour change systems, and so on.

The invention will now be illustrated by the following non-limiting Examples.

### Example

The objective of the experiment was to assess the impact of cleaning teeth on psychological benefits and the impact of these benefits as detected by third party evaluators.

Sixty-eight participants (35 females, 33 males) refrained from brushing their teeth and all forms of oral hygiene that morning. Participants were instructed to wear black T-shirts. After participants had put their t-shirt on they were all asked to rate their own attractiveness and confidence (before brushing). The brushed group then brushed their teeth for one minute with 1 g Closeup gel (2014 ex Brazil) toothpaste and immediately after brushing rated their own attractiveness and confidence a second time before conducting the video task. The not brushed group completed the video task and then rated their attractiveness and confidence for a second time.

### Video Task

Participants were asked to prepare and to describe identical scenarios. They were filmed using identical conditions during the preparation and when describing the scenario. Video clips were edited to a duration of 10 s which was taken half-way through the 30 s clip; all video clips were edited at the same time point. There were two videos for each participant, a 10 second preparation and a 10 second talk video.

Forty-eight (25 female, 23 male) evaluators watched the muted video clips. Evaluators rated the muted videos for attractiveness, confidence and as an observer 'I can understand why others would like to (a) meet that person, (b) go out for dinner with that person, (c) date that person and (d) kiss that person. The six attributes rated were randomised following each video to ensure that evaluators attended to the question and task.

The ratings were averaged across all evaluators to provide a dataset which consisted of one data point per participant for each combination of Attribute (confidence, attractiveness, date, dinner, kiss, meet) Grooming (brushing or not brushing) and Phase (prep or talk) prior to statistical analysis. Mixed-model analysis of variance with averaged third party evaluator attribute scores as the dependent variable was performed using the PROC MIXED procedure in SAS, fitting as fixed effects: participant gender, grooming, phase and all 2/3 factor interactions between them, and participant ID as a random effect. Each Attribute was analysed separately.

### Results

There was a main effect of Phase for all Attributes; confidence [F(1,64)=362.2, p<0.0001], attractiveness [F(1,64)=104.4, p<0.0001], date [F(1,64)=213.5, p<0.0001], dinner [F(1,64)=296.9, p<0.0001], kiss [F(1,64)=155.6, p<0.0001] and meet [F(1,64)=293.8, p<0.0001]. Goal directed behaviour (talk phase) that is observed by others are rated higher than when passively sitting generally looking down with little eye contact while thinking about future actions (prep phase). Hence, goal directed action has general positive effects on how the person is perceived. There was a main effect of Grooming for all Attributes; confidence [F(1,64)=19.7, p<0.0001], attractiveness [F(1,64)=6.5, p=0.013], date [F(1,64)=7.8, p=0.007], dinner [F(1,64)=8.3, p=0.005], kiss [F(1,64)=7.4, p=0.008] and meet [F(1,64)=6.9, p=0.011].

The assessment demonstrated that cleaning the teeth with toothpaste has positive effects on nonverbal behaviour such that the brushed individuals appear more confident, attractive etc. to others. There was no main effect of Gender for any Attribute. A two-way interaction of Phase x Gender for confidence [F(1,64)=4.9, p=0.031] and date [F(1,64)=5.0, p=0.029].

These main effects were superseded by a significant three-way interaction between Grooming x Phase x Gender for all Attributes; confidence [F(1,64)=7.7, p=0.007] attractiveness [F(1,64)=6.3, p=0.014], date [F(1,64)=7.1, p=0.010], dinner [F(1,64)=7.6, p=0.008], kiss [F(1,64)=7.9, p=0.006] and meet [F(1,64)=7.7, p<0.007].

For females a significant benefit for tooth brushing was found during the preparation phase [e.g. for confidence t(100)=2.7, p=0.009]; the average increase in score due to brushing across all attributes was 15 units. Whereas for males a significant benefit for tooth brushing was found during the talk phase [e.g. for confidence t(100)=4.7, p<0.0001]; the average increase in score due to brushing across all attributes was 21 units.

### Eye Gaze

FaceReader software provided a classification of eye gaze direction (either 'Forward', 'Left', 'Right' or 'Unknown') at 40 ms intervals. The total number of 'Forward' ratings was expressed as a percentage of 'Forward' + 'Left' + 'Right' ratings, for each 10 s video clip. This percentage measure was used in the subsequent analysis and specifically to compare the brushed and not brushed groups within each of the phases. An unpaired Student's t-test showed that '%Forward' was significantly higher for the brushed group compared to the not brushed group in the talk phase (t=3.98, p<0.001), but not for the prep phase. The results are shown in Table 1 and Table 2 below.

**Table 1**

| | Talk Phase | Eye Gaze |
|---|---|---|
| Female | Brushed | 87.89 ± 2.85 |
| | Not Brushed | 73.29 ± 5.43* |
| Male | Brushed | 87.62 ± 3.9 |
| | Not Brushed | 67.06 ± 6.48** |

**Table 2**

| | Prep Phase | Eye Gaze |
|---|---|---|
| Female | Brushed | 58.41 ± 6.2 |
| | Not Brushed | 60.5 ± 9.5 |
| Male | Brushed | 63.34 ± 8.59 |
| | Not Brushed | 54.59 ± 8.12 |

## Claims

1. Use of an oral care composition to enhance the behaviours associated with attractiveness of an individual.

2. Use according to any preceding claim in which the oral care composition is used within one hour of the behaviours associated with attractiveness of an individual.

3. Use according to any preceding claim in which the behaviour associated with attractiveness is non-verbal.

4. Use according to any preceding claim in which the breath is not assessed

5. Use according to any previous claim in which the behaviour is assessed by a third party

6. Use according to any preceding claim in the behaviour associated with attractiveness which increased eye gaze.

7. Use according to any previous claim in which the composition is a toothpaste.

8. Use according to any preceding claim in which the toothpaste is a gel toothpaste.

9. Use according to any preceding claim in which the composition comprises an abrasive.
